(19) Europäisches Patentamt
European Patent Office
Office européen des brevets

(11) Publication number: **0 209 643 B1**

# (12) EUROPEAN PATENT SPECIFICATION

(45) Date of publication of patent specification: **08.07.92**

(51) Int. Cl.5: **A61K 37/02**, G01N 33/569, A61K 39/015

(21) Application number: **86105223.1**

(22) Date of filing: **16.04.86**

(54) **Peptide composition useful for the malaria vaccine manufacture as well as for the preparation of diagnostic kits for the detection of malarial diseases.**

(30) Priority: **25.07.85 IT 2171885**

(43) Date of publication of application:
**28.01.87 Bulletin 87/05**

(45) Publication of the grant of the patent:
**08.07.92 Bulletin 92/28**

(84) Designated Contracting States:
**AT BE CH DE FR GB IT LI LU NL SE**

(56) References cited:
**EP-A- 0 166 410**
**EP-A- 0 191 748**
**EP-A- 0 192 626**
**WO-A-86/05790**

**SYNTHETIC PEPTIDES, vol. 4, G.R. PETTIT, Elsevier Scientific Publishing Company 1976, Amsterdam-Oxford-New York**

**CHEMICAL ASTRACTS, vol. 103, no. 7, 19th August 1985, page 493, column 2, abstract no. 69462q, Columbus, Ohio, US; F. ZAVALA et al.: "Rationale for development of a synthetic vaccine against Plasmodium falciparum malaria."**

**Science, vol. 228, p. 1436-1440 (1985)**

**NATURE, vol. 285, p. 664 (1980)**

(73) Proprietor: **ENIRICERCHE S.p.A.**
**Corso Venezia 16**
**I-20121 Milan(IT)**

(72) Inventor: **Verdini, Antonio Silvio**
**Via S. Martino, 12**
**I-00015 Monterotondo(IT)**
Inventor: **Pinori, Massimo**
**Via Tevere, 18**
**I-00015 Monterotondo(IT)**

(74) Representative: **Cioni, Carlo et al**
**c/o ENIRICERCHE S.p.A. BRELID Via F. Maritano 26**
**I-20097 San Donato Milanese(IT)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).



Rank Xerox (UK) Business Services

## Description

The present invention relates to a peptide composition useful for the malaria vaccine manufacture as well as for the preparation of diagnostic kits for the detection of malarial diseases having the following general formula:

H-(Asn-Ala-Asn-Pro)$_n$-OH (I)

wherein Asn is Asparagine
Ala is Alanine
Pro is Proline
and wherein n is equal to or higher than 10.

Plasmodium are hemoparasites having a life cycle alternating between an invertebrate host, wherein they exhibit a sexual reproduction, and a vertebrate host wherein they multiply by simple schizogony.

In the human body Plasmodium gives rise to malaria, a serious and very wide-spread disease.

Four species are pathogenic for the human organism: Plasmodium vivax, Plasmodium malarial, Plasmodium ovalis and Plasmodium falciparum.

Among them Plasmodium falciparum is the etiological agent of the most serious disease, the so called malignat tertian. The malarial parasite is transmitted by the bite of anopheles mosquito, which is the invertebrate host, into the human body and there multiplies in the cells of the reticularendothelial and hepatic system. Such a first phase is followed, after about one week, by a second phase in which the parasite begins to invade the blood stream, to infect the erythrocytes and to divide.

Some of the above parasites do not follow the schizogonic cycle and transform into gametocytes.

The female mosquito by bitting a malarial man, having Plasmodium in the blood stream, together with the infected blood, receives also the gametocytes which, by a sexual reproduction cycle, give rise to thin and tapering elements called sporozoites.

Said sporozoites, through the bite of the mosquito are again trasmitted to the human organism in which they begin a new schizogonic cycle.

Vaccines have been developed for each phase of the life cycle of the malarial Plasmodium.

A particular interest presents the development of vaccines against sporozoites which, if effective for the human organism, may prevent the subsequent stages responsible for the disease and the trasmission thereof.

The possibility to immunize and protect human and animal organisms against malarial sporozoites has been studied by R.W. Nussenzweig et al. Mil. Med. 134, 1376 (1969) by using irradiated sporozoites.

Furthermore it has been found that such a protection is correlated with the production of specific antibodies for the protein present on the sporozoite surface, called circumsporozoite protein (CS).

Recently Dame et al. Science 225, 593-599 (1984) cloned and sequenced the gene coding the CS protein of Plasmodium falciparum.

Said protein, consisting of 412 aminoacids, has a central repeat domain composed of 37 tetrapeptides with the sequence Asn-Ala-Asn-Pro and 4 tetrapeptides with the sequence Asn-Asp-Val-Pro.

A study carried out by using monoclonal antibodies specific for the CS protein has shown in this domain the presence of specific epitopes, therefore confirming the immunodominant property of this domain and the possibility to use it as a basis for the preparation of vaccine against malaria. Recently, W. Ripley Ballon et al. [Science,228, 996-999 (1985)] have synthetized peptides consisting of one to three amino acid quadruplets of the CS protein domain of the Plasmodium falciparum sporozoite.

The authors have found that, said peptides when linked to a carrier protein by an amino terminal cysteine residue, are able to induce a positive antibody response in experimental animals.

Furthermore the produced antibodies recognize the native circumsporozoitic protein and inhibit in vitro the sporozoitic infection of the human liver cells.

However the use of said synthetic peptides for the preparation of vaccines against the Plasmodium falciparum sporozoites presents some limitations.

The reasons of said limitations reside mostly on

- the limited amount of peptides that can link to the carrier protein;
- the host sensibilization which may induce a fast peptide elimination;
- the epitope-specific suppression and finally;
- the necessity of repeated inocula where the immunity against Plasmodium falciparum is of short duration.

According to J.F. Young et al. [Science 228, 958-962 (1985)] proteins consisting of 16, 32 or 48 repeating aminoacid quadruplets of the domain present in the CS protein of P. falciparum fused to a 32 amino acid peptidic segment, are expressed in E. coli.

The obtained proteins, tested on experimental animals, produce a high antibody title, and the induced antibodies recognize the native protein and block in an in-vitro assay the sporozoite invasion of the tumoral hepatic cells.

However by operating according to Young et al. in the above reference, it appears that it is first necessary to obtain a preliminary accurate evaluation of the possible risks connected to the use in human therapy of products obtained through genetic manipulation, and then to have some difficulties in producing and purifying the desired proteins.

E. coli is a pathogenic bacterium and its use in a fermentation process to produce heterologous proteins requires accurate controls.

Moreover the E. coli synthesized products remain in the cell and their extraction requires the breaking or lysing of the microorganisms.

The extracted products must be subsequently removed and purified.

In short such a process is complicated by the many steps required and low overall yields that make the process unattractive from an industrial point of view.

It has been now found that it is possible to overcome the drawbacks of the prior art by using a peptide composition useful for the malaria vaccine manufacture as well as diagnostic kits for the detection of malarial diseases, which can be obtained in a pure form with a simple and economic process.

It is therefore an object of the present invention to provide a peptide composition useful in the manufacture of a vaccine against malaria, as well a diagnostic kit to detect malarial diseases, consisting of a peptide mixture having the formula:

H-(Asn-Ala-Asn-Pro)$_n$-OH (I)

wherein:
Asn is Asparagine, Ala is Alanine, Pro is Proline and n is comprised from 10 to 100 and wherein at least 60% weight of the peptides has n ranging from 37 to 41, obtained by a process characterized in that:

a) a tetrapeptide is synthesized with the Asn terminal amino group protected having the following formula:

X-Asn-Ala-Asn-Pro-OH (II)

wherein X is an acidolabile protecting group;

b) tetrapeptide (II) is activated by reaction with halogenated phenol derivatives to obtain the active ester of said tetrapeptide at the terminal carboxyl group of Pro

X-Asn-Ala-Asn-Pro-OY (III)

wherein X has the above meaning and Y is is the halogenated phenol derivative radical;

c) the amino protecting group is removed from said tetrapeptide (III) by acidolysis to produce the tetrapeptide

HCl.H-Asn-Ala-Asn-Pro-OY (IV)

wherein Y has the above meaning;

d) the tetrapeptide (IV) is polycondensed in the presence of an organic catalyst of basic nature selected among the tertiary alkyl amines wherein the alkyl group consists of 1-4 carbon atoms;

e) the peptide mixture is recovered having the formula:

H-(Asn-Ala-Asn-Pro)$_n$-OH (I)

wherein n has the above meaning.

It is another object of the present invention to provide a process for the preparation of said peptide composition.

A further object of the present invention is the use of said peptide composition to manufacture malaria vaccine as well as diagnostic kits to detect malarial diseases.

Other objects of the present invention will be evident from the following specification and experimental examples.

More particularly the peptide composition, according to the present invention, consists of a peptide mixture according to the following formula:

H-(Asn-Ala-Asn-Pro)$_n$-OH (I)

wherein Asn is Asparagine
Ala is Alanine
Pro is Proline
and wherein n is equal to or higher than 10.

According to the present invention the above peptide mixture may be prepared by a process comprising:

a) the synthesis of a tetrapeptide protected at the terminal amino group of Asn having the following formula

X-Asn-Ala-Asn-Pro-OH (II)

wherein X is an acidolabile protecting group

b) the activation of tetrapeptide (II) by reaction with halogenated phenol derivatives to obtain the active ester of said tetrapeptide at the terminal carboxylic group of Pro

X-Asn-Ala-Asn-Pro-OY (III)

wherein X has the above meaning and Y is the halogenated phenol derivative radical

c) the removal of the amino protective group from said tetrapeptide (III) by acidolysis to produce tetrapeptide

HCl.H-Asn-Ala-Asn-Pro-OY (IV)

d) the policondensation of said tetrapeptide (IV) in the presence of an organic catalyst of basic nature, which is a tertiary alkylamine having $C_{1-4}$ alkyl groups

e) the recovery of the peptide mixture

H-(Asn-Ala-Asn-Pro)$_n$-OH (I)

wherein n is 10 to 100.

Step a)

In the step a) of the process according to the present invention, the preparation of the tetrapeptide protected at the terminal amino group of Asn having the formula

X-Asn-Ala-Asn-Pro-OH (II)

is carried out by homogeneous phase condensation by a technique well known in peptide synthesis.

Generally the aminoacids conveniently protected at the amino or carboxylic function are dissolved in an inert (non reactive) solvent in the presence of condensation agents selected among those known in the art.

Suitable organic solvents are chlorinated aliphatic hydrocarbons, aliphatic ketones, alkyl esters.

Specific examples of said solvents are: N, N' dimethylformamide, chloroform, ethyl acetate, tetrahydrofuran.

The amino protecting groups are selected among the acidolabile ones, i.e. that can be removed by acid hydrolysis.

Particularly preferred is tert-butyloxycarbonyl (Boc).

The reaction temperature of the condensation may generally vary from -10°C to +40°C and the corresponding reaction time is the one necessary to complete or substantially complete the reaction.

Step b)

According to the present invention tetrapeptide (II) protected at the amino terminal group is activated by reacting with a halogenated phenol derivative to produce its active ester at the terminal carboxyl group of Pro:

X-Asn-Ala-Asn-Pro-OY (III)

where X has the above meaning and Y is the halogenated phenol derivative radical.

Halogenated phenol derivatives useful in the process according to the present invention are fluorinated or chlorinated derivatives.

Particularly useful are pentachlorophenol, trichlorophenol and pentafluorophenol.

The Pro carboxyl group activation reaction is carried out by contacting the tetrapeptide (II), after removal of the protecting group from the Pro carboxyl group, with the halogenated phenol derivative, in a relative molar ratio of about 1 to 1, the reaction takes place in an inert organic solvent at a temperature from -10°C to +40°C.

More particularly the reaction takes place at room temperature or near thereto.

Examples of suitable organic solvents are the aprotic ones such as ethyl acetate or chlorinate aliphatic hydrocarbons.

The obtained solution is cooled to a temperature of about 0°C and then reacted with a condensation agent in a molar ratio between condensation agent and one or other starting reagent equal to or higher than 1:1.

More particularly as condensation agent dicyclohesylcarbodiimide is used. The resulting mixture is kept at a temperature from -10°C and +40°C for a period of 15 minutes to 4 hours.

In practice it is advisable to operate at a temperature of about 0° for a period of time of 1 hour and at room temperature (20-25°C) or near thereto for a corresponding period of 1 hour.

By operating within the preferred conditions tetrapeptide (III) is obtained having a melting point of 160°-164°C and a $[\alpha]_D^{22}$ of -74.8° (c 0.75; methanol).

Step c)

In this step the amino protecting group is removed from said activated tetrapeptide (III) by acid hydrolysis.

The acidolysis reaction is carried out by using trifluoroacetic or concentrated hydrochloric acid at room temperature for about 1 hour.

In step d) of the process according to the present invention said activated tetrapeptide (IV), after removal of the protecting group, is polycondensed in the presence of an excess quantity of an organic catalyst of basic nature, selected from among tert-alkyl-amines wherein alkyl group consists of 1 to 4 carbon atoms.

The preferred amine is triethylamine.

According to the present invention the reaction takes place in liquid phase in an inert organic solvent.

Suitable organic solvents are dimethylsulphoxide, dimethylformamide, hexamethylphosphoramide.

The reaction temperature may vary from -15°C to +40°C about and the corresponding reaction time may vary from 24 hours to 4 days.

In pratice the reaction is carried out at room temperature or near thereto. In such a case the time required to complete or to substantially complete the reaction is roughly 72 hours.

At the end of the reaction a peptide composition is obtained from which a peptide mixture is recovered, by gel chromatography, having the formula

H-(Asn-Ala-Asn-Pro)$_n$-OH

wherein n is from 10 to 100.

Said mixture may be utilized as such to manufacture malaria vaccines or diagnostic kits to detect malarial diseases.

According to another embodiment of the present invention such a mixture may be fractioned to obtain mixture having a narrower molecular weight (MW) distribution.

The fractionation is carried out by gel chromatography by using a 2.5 X 80 cm column with a Sephadex (R) G50 filler, at a temperature of 20-25 °C and with a flow rate of 0.5 ml/minute. By operating in such a way fractions having a molecular weight (MW) of about 16000 in which n is between 37 and 41 and fractions having a molecular weight (MW) of about 8000 corresponding to a value of n of 20-3 are separated and recovered.

Particularly suitable are peptide mixtures having MW of about 16,000 and n values between 37 to 41.

Both the overall mixture and the single fractions show a biological activity in mice.

Particularly active is the peptide mixture having n from 37 to 41. Therefore all such mixtures may be used to manufacture malaria vaccines as well as diagnostic kits to detect malarial diseases.

The following experimental examples will illustrate the invention.

## Example 1

### a) Synthesis of the benzyl ester of butyloxycarbonyl-asparaginyl-proline (boc-Asn-Pro-OBz)

In a 250 ml reaction flask, fitted with stirrer, 10 g (42.5 mmole) of HCl.H-Pro-OBz and 150 ml of N,N'dimethylformamide were introduced. The mixture was stirred and a solution was obtained.

7.1 ml (45 mmole) of diisopropylethylamine (DIPEA) and 8.4 g (62 mmole) of N-hydroxybenzotriazole (HOBt) were added under stirring to said solution and the solution was cooled to 0 °C. To said cooled solution 9.37 g (45 mmole) of dicyclohexylcarbodiimide (DCI) were added. The condensation reaction was carried out keeping the solution temperature of 0 °C for a period of 90 minutes.

Thereafter the solvent was evaporated from the reaction mixture, the residue was dissolved using 200 ml of ethyl acetate (EtoAc) and washed successively with 30 ml of a 5% w/v sodium bicarbonate solution, 30 ml of a 10% w/v citric acid solution and then 30 ml of a sodium chloride saturated solution.

The organic phase was separated from the solution and dryed with about 10 g of anhydrous $MgSO_4$.

The solvent was then evaporated from the solution and the residue was recovered by crystallization from 100 ml of EtOAC/n-hexane (1/1 v/v).

12 g of benzyl ester of tert-butyloxycarbonyl asparaginyl proline were obtained having a melting point of 105 °-106 °C and a $[\alpha]_D^{22}$ of -83.6 ° (c 1.2; methanol).

### b) Synthesis of benzyl ester of tert-butyloxycarbonyl-alanylasparaginyl proline (Boc-Ala-Asn-Pro-OBz)

117 g (28 mmole) of Boc-Asn-Pro-OBz obtained according to step a) were added to 200 ml of a BtOAc solution containing 4 N HCl.

The stirred solution was kept at room temperature (20-25 °C) for 1 hour about.

At the end of the acidolytic reaction the solvent was evaporated from the reaction mixture and the residue was triturated with diethyl ether to obtain a white solid product.

Said white solid together with 5.7 g (30 mmole) of Boc-Ala-OH, 5.5 g (41 mmole) of HOBt and 3.08 ml (30 mmole) of N-methylmorpholine (NMM) were added to 100 ml of DMF. The solution was cooled to 0 °C and then 6.3 g (30 mmole) of HCl were added thereto. The reaction was carried out at 0 °C for 90 minutes.

At the end of such a period the solvent was completely evaporated from the reaction mixture; the residue was dissolved in 200 ml of EtOAc and then was extracted by subsequent washing in the same way as reported in step a). At the end the organic phase was separated and dried over anhydrous Mg $SO_4$.

The solvent was separated from the organic phase and the residue was recovered by trituration with n-hexane.

10 g (73% yield) of benzyl ester of tert-butyloxycarbonylalanylasparaginylproline were obtained having melting point 71-72 °C and $[\alpha]_D^{22}$ of -94.7 ° (c 1.5: methanol).

### c) Synthesis of benzyl ester of tert-butyloxy carbonylasparaginylalanyl asparaginyl proline (Boc-Asn-Ala-Asn-Pro-OBz)

The synthesis was carried out in the same way as in the above step a) using 9.9 g (20 mmole) of Boc-Ala-Asn-Pro-OBz.

At the end of the acidolytic reaction, the residue was dissolved in 100 ml of DMF containing 5.1 g (22 mmole) of Boc-Asn-OH.

The condensation reaction was carried out by keeping the solution at a temperature of 0 °C for a period of time of 2 hours about.

Then the procedure of step a) was followed.

6 g (yield 50%) of benzyl ester of tert-butyloxy carbonilasparaginylalanylasparaginylproline were obtained having a melting point of 153-154 °C and $[\alpha]_D^{22}$ of -91.1 ° (c 0.9, methanol).

Example 2

Synthesis of the pentachlorophenolic ester of tert-butyl-oxycar bonylasparaginylalanylasparaginyl-proline (Boc-Asn-Ala-Asn-Pro-OPCP)

In a 200 ml reaction vessel, fitted with stirrer, 50 ml of methanol were introduced as well as 1,5 g (2,5 mmole) of Boc Asn-Ala-Asn-Pro-OBz and 600 mg (9.5 mmole) of ammonium formiate.

To said solution kept under a nitrogen atmosphere and stirred, 1 g of palladium on coal (10%) catalyst was added.

From the reaction mixture kept under stirring a suspension was obtained.

Said stirred suspension was kept under a nitrogen atmosphere and at room temperature up to a complete removal of the protecting benzyl group from proline carboxylic group. At the end of the reaction, the catalyst was removed by filtration from the suspension and the solvent was evaporated undervacuum up to dryness. Said residue was dissolved in 50 ml of EtOAc containing 670 mg (2,5 mmole) of pentachlorophenol.

The solution was cooled to 0 °C and 520 mg (2,5 mmole) of HCl were added thereto.

The stirred solution was kept at 0 °C for 1 hour and then at room temperature for a period of 1 hour about.

At the end of such a period the obtained dicyclohexylurea was recovered by filtration and the solvent was completely evaporated.

The residue was treated at 70 °C with 100 ml of isopropyl alcohol and then diethyl ether was added thereto up to incipient crystallization. Thus, there was obtained 1 g of the desired product (pentachlorophenolic ester of tert-butyloxycarbonyl asparaginylalanylasparaginylproline having a melting point of 160-164 °C and $[\alpha]_D^{22}$ of -74.8 °C (c 0.75, methanol).

Example 3

Synthesis of polyasparaginylalanylasparaginyl proline H-(Asn-Ala-Asn-Pro)$_n$-OH

500 mg (0.65 mmole) of the Boc-Asn-Ala-Asn-Pro-OPCP obtained in example 2 were dissolved in 2.0 ml of trifluoracetic acid. The solution was stirred and kept at room temperature for 1 hour about.

At the end of the reaction, the solvent was evaporated under vacuum and the oily residue was tritured with diethylether until a white solid was obtained.

The product was suspended in 2 ml of dimethyl-sulphoxide and the mixture was stirred to obtain a solution. To said solution, kept under stirring, 300 ml of triethylamine were added in small proportions.

The solution was kept at room temperature for 24 hours and, after addition of 100 ml of triethylamine, for other 48 hours.

At the end of the reaction the solution was added dropwise, in a period of 5 minutes about, to 350 ml of anyhydrous ethanol kept under mild stirring.

The obtained precipitate was removed from the suspension by filtration and dried under vacuum.

The product was divided into fractions of 30 mg each about, each fraction was dissolved in 1 ml of 0.1 N acetic acid and subyected to chromatography separation.

A 2.5 X 80 mm Sephadex(R) G25 FINE (Farmacia Upsala) column was used at a temperature of 20-25 °C and a flow rate of 0.5 ml/minute.

The fractions were collected at intervals of 12 minutes. The fractions from 26 to 39 were gathered together and lyophilized.

115 mg (yield 40%) of polymer were obtained consisting of a mixture of H-(Asn-Ala-Asn-Pro)$_n$-OH where n is equal to or higher than 10.

Said polymer was then fractioned by gel chromatography operating in the same way as above using a column filled with Sephadex(R) G50.

At the end 30 mg of polymer were obtained having an average molecular weight of 16.000, corresponding to an n of 37 to 41 as well as 75 mg of polymer having a molecular weight lower than 16.000.

The molecular weight of the fractions having n from 37 to 41 was confirmed by chromatography on a 1,5 X 100 cm column filled with BIOGEL(R) A-5M (BIORAD) equilibrated with 6M guanidinium chloride using as internal calibration standards albumine (MW 45,000), myoglobin (MW 18,000), trypsin (MW 8,000) and tryptophan (MW 200).

The operation was performed at room temperature with a flow rate of 2,5 ml/hour and collecting the fractions every hour.

Example 4

ELISA assay for detection of anti-sporozoites antibodies of Plasmodium falciparum using a synthetic peptide as substrate

a) Preparation of synthetic peptide coated plates.

The fraction of synthetic peptides with n = 37 - 41, was suspended in distilled water and then, aliquots of said suspension mantained at -70 °C, were diluted in a phosphate buffered saline (PBS) (pH 7.8) at final concentration of 1 µg/ml.

100 µl of said solution were transferred into microtiter plates, communely used in the ELISA procedure.

The microtiter plates were incubated overnight, at room termperature (20-25 °C) in a wet room.

b) Test

At the end of the incubation, the plates were washed 4 times with PBS containing 0,05% by wt. of Tween 20 (PBS-T) and then saturated with 200 µl of PBS-T containing 5% by wt. of powdered lipid-free milk (PBS-T-M).

The plates were left to stand for about 1 hour at room temperature.

After removal of the saturated solution, 100µl of serum samples to be tested, suitably diluted in PBS-T-M 2,5%, were added in each well of the microtiter plates.

The plates were incubated for about 1 hour at room temperature.

Then the plates were washed 4 times with PBS-T and added with 100 µl of peroxidase linked antiserum (IgG fraction from goat) against IgG, IgM or the whole human or animal immunoglobulin fraction.

The antiserum was suitably diluted in 2,5% PBS-T-M.

After incubation for 1 hour at room temperature, the plates were washed 4 times with PBS-T.

Then 100 µl of a solution of orto-phenilendiammine in 0,1M citrate buffer (0,4 mg/ml) containing 0,01% of $H_2O_2$ were added to the plates.

The enzymatic reaction was stopped 20 minutes later by adding 2,5 N $H_2SO_4$.

The optical density in each well was determinated by absorbance measuring at 492 nm using a micro ELISA (Multiskan Titertek).

The results were expressed as the difference between the optical densities of coated and uncoated plates (figura 1).

Figura 1 - in abscissa were reported serum dilution

●—● : patient from Surinam

○—○ : patient from Zaire

△—△ : Caucasian living for 6 years in Ivory Coast

■—■ : healthy normal control

in ordinate Absorbance 492 mm.

## Claims

**1.** A peptide composition useful in the manufacture of a vaccine against malaria, as well a diagnostic kit to detect malarial diseases, consisting of a peptide mixture having the formula:

H-(Asn-Ala-Asn-Pro)$_n$-OH (I)

wherein:

Asn is Asparagine, Ala is Alanine, Pro is Proline and n is from 10 to 100 and wherein at least 60% weight of the peptides has n ranging from 37 to 41, obtained by a process characterized in that:

a) a tetrapeptide is synthesized with the Asn terminal amino group protected having the following formula:

X-Asn-Ala-Asn-Pro-OH (II)

wherein X is an acidolabile protecting group;

b) tetrapeptide (II) is activated by reaction with halogenated phenol derivatives to obtain the active ester of said tetrapeptide at the terminal carboxyl group of Pro

X-Asn-Ala-Asn-Pro-OY (III)

wherein X has the above meaning and Y is is the halogenated phenol derivative radical;

c) the amino protecting group is removed from said tetrapeptide (III) by acidolysis to produce the tetrapeptide

HCl.H-Asn-Ala-Asn-Pro-OY (IV)

wherein Y has the above meaning;

d) the tetrapeptide (IV) is polycondensated in the presence of an organic catalyst of basic nature selected from among the tertiary alkyl amines wherein the alkyl group consists of 1-4 carbon atoms;

e) the peptide mixture is recovered having the formula:

H-(Asn-Ala-Asn-Pro)$_n$-OH (I)

wherein n has the above meaning.

**2.** Process to obtain a peptide composition according to claim 1, wherein in step a) the amino protecting group is Boc.

**3.** Process to obtain a peptide composition according to claim 1, wherein in step b) the halogenated phenol derivatives are selected among the fluorinated and chlorinated derivatives.

**4.** Process according to claim 3, wherein the halogenated phenol derivatives are pentachlorophenol, trichlorophenol and pentafluorophenol.

**5.** Process to obtain a peptide composition according to claim 1, wherein in step b) the molar ratio between tetrapeptide (II) and halogenated phenol derivative is equal or nearly equal to 1/1 and the reaction is carried out in liquid phase in an inert organic solvent at a temperature from -10° to 40°C.

**6.** Process according to claim 5, wherein the organic solvent is ethyl acetate.

**7.** Process according to claim 5, wherein the temperature is comprised between 0° and 25°C.

**8.** Process to obtain a peptide composition according to claim 1, wherein in step c) the amino protecting group is removed by acidolysis with trifluoroacetic or hydrochloric acid, at room temperature (20-25°C) for a period equal or nearly equal to 1 hour.

**9.** Process to obtain a peptide composition according to claim 1, wherein in step d) the tertiary alkyl amine is triethylamine.

**10.** Process according to claim 9, wherein the polycondensation is carried out at room temperature (20-25°C) or near thereto.

**11.** Process to obtain a peptide composition according to claim 1, wherein in step e) the separation is performed by gel chromatography.

**12.** Use of the composition according to claim 1, for the manufacture of malaria vaccine.

**13.** Use of the composition according to claim 1, for the manufacture of diagnostic kits to detect malarial diseases.

**14.** A method for the detection of antibodies specific for Plasmodium falciparum sporozoites in human blood by ELISA assay using as substrate a synthetic peptide composition according to claim 1.

**Revendications**

**1.** Composition peptidique utilisable pour la préparation d'un vaccin contre la malaria, ainsi qu'une trousse de diagnostic pour détecter les affections malariennes, constituée par un mélange de peptides répondant à la formule :

H-(Asn-Ala-Asn-Pro)$_n$-OH (I)

dans laquelle :
Asn représente l'Asparagine, Ala représente l'Alanine, Pro représente la Proline et n vaut de 10 à 100, et dans laquelle, dans au moins 60 % en poids des peptides, n vaut de 37 à 41, mélange obtenu par un procédé caractérisé en ce que :

a) un tétrapeptide ayant la formule suivante est synthétisé, le groupe amino termina! d'Asn étant protégé :

X-Asn-Ala-Asn-Pro-OH (II)

dans laquelle X est un groupe protecteur acidolabile,
b) le tétrapeptide (II) est activé par réaction avec un dérivé halogéné de phénol, ce qui donne un ester actif dudit tétrapeptide, formé avec le groupe carboxyle terminal de Pro

X-Asn-Ala-Asn-Pro-OY (III)

formule dans laquelle X a la signification mentionnée ci-dessus et Y est le reste de dérivé halogéné de phénol,
c) le groupe amino-protecteur est éliminé dudit tétrapeptide (III) par acidolyse, ce qui donne le tétrapeptide

HCl.H-Asn-Ala-Asn-Pro-OY (IV)

dans lequel Y a la signification mentionnée ci-dessus,
d) le tétrapeptide (IV) est polycondensé en présence d'un catalyseur organique à caractère basique, choisi parmi les alkylamines tertiaires dans lesquelles le groupe alkyle comprend 1 à 4 atomes de carbone,
e) le mélange de peptides répondant à la formule :

H-(Asn-Ala-Asn-Pro)$_n$-OH (I)

dans laquelle n a la signification mentionnée ci-dessus, est récupéré.

**2.** Procédé pour obtenir une composition peptidique selon la revendication 1, dans lequel dans l'étape a), le groupe amino-protecteur est le groupe Boc.

**3.** Procédé pour obtenir une composition peptidique selon la revendication 1, dans lequel, dans l'étape b), le dérivé halogéné de phénol est choisi parmi les dérivés fluorés ou chlorés.

**4.** Procédé selon la revendication 3, dans lequel les dérivés halogénés de phénol sont le pentachlorophénol, le trichlorophénol et le pentafluorophénol.

**5.** Procédé d'obtention d'une composition peptidique selon la revendication 1, dans lequel, dans l'étape b), le rapport molaire du tétrapeptide (II) au dérivé halogéné de phénol est égal ou presque égal à 1/1 et la réaction est réalisée en phase liquide, dans un solvant organique inerte, à une température de -10 ° C à 40 ° C.

**6.** Procédé selon la revendication 5, dans lequel le solvant organique est l'acétate d'éthyle.

**7.** Procédé selon la revendication 5, dans lequel la température est comprise entre 0 ° et 25 ° C.

**8.** Procédé d'obtention d'une composition peptidique selon la revendication 1, dans lequel, dans l'étape c), le groupe amino-protecteur est éliminé par acidolyse à l'aide de l'acide trifluoroacétique ou, chlorhydrique, à la température ambiante (20-25 ° C), en une durée égale ou presque égale à 1 heure.

**9.** Procédé pour l'obtention d'une composition peptidique selon la revendication 1, dans lequel, dans l'étape d), l'alkylamine tertiaire est la triéthylamine.

**10.** Procédé selon la revendication 9, dans lequel la polycondensation est réalisée à la température ambiante (20-25 ° C) ou à une température voisine de celle-ci.

**11.** Procédé d'obtention d'une composition peptidique selon la revendication 1, dans lequel, dans l'étape e), la séparation est réalisée par chromatographie sur gel.

**12.** Utilisation de la composition selon la revendication 1, pour la préparation d'un vaccin contre la malaria.

**13.** Utilisation de la composition selon la revendication 1, pour la préparation de trousses de diagnostic pour détecter les affections malariennes.

**14.** Procédé de détection d'anticorps spécifiques aux sporozoïtes de Plasmodium falciparum dans le sang humain, par un test ELISA dans lequel on emploie comme substrat une composition peptidique synthétique selon la revendication 1.

## Patentansprüche

**1.** Peptidzusammensetzung, die zur Herstellung einer Vakzine gegen Malaria sowie als ein Diagnosekit zur Feststellung von Malaria-Erkrankungen geeignet ist und aus einem Peptidgemisch mit der Formel:

H-(Asn-Ala-Asn-Pro)$_n$-OH (I)

besteht, worin:
Asn für Asparagin, Ala für Alanin und Pro für Prolin steht und n einen Wert von 10 bis 100 aufweist und worin wenigstens 60 Gew.-% der Peptide Werte für n im Bereich von 37 bis 41 aufweisen, welches Gemisch nach einem Verfahren erhalten wird, das sich dadurch auszeichnet, daß:

a) ein Tetrapeptid synthetisiert wird, worin die endständige Asn-Aminogruppe geschützt ist und das Tetrapeptid die folgende Formel aufweist:

X-Asn-Ala-Asn-Pro-OH (II),

worin X eine säurelabile Schutzgruppe darstellt;

b) das Tetrapeptid (II) durch Umsetzung mit halogenierten Phenolderivaten aktiviert wird, um den aktiven Ester dieses Tetrapeptids an der endständigen Carboxylgruppe von Pro zu erhalten:

X-Asn-Ala-Asn-Pro-OY (III),

worin X die vorstehend genannte Bedeutung aufweist und Y den halogenierten Phenolderivatrest bezeichnet;

c) aus dies Tetrapeptid (III) durch Acidolyse die Aminoschutzgruppe entfernt wird, um das Tetrapeptid

HCl.H-Asn-Ala-Asn-Pro-OY (IV)

auszubilden, worin Y die obige Bedeutung hat;

d) das Tetrapeptid (IV) in Anwesenheit eines organischen Katalysators mit basischer Natur, ausgewählt unter tertiären Alkylamien,

worin die Alkylgruppe aus 1 bis 4 Kohlenstoffatomen besteht, polykondensiert wird;
e) das Peptidgemisch mit der Formel

H(Asn-Ala-Asn-Pro)$_n$-OH (I)

gewonnen wird, worin n die obige Bedeutung hat.

**2.** Verfahren zur Gewinnung einer Peptidzusammensetzung nach Anspruch 1, worin in Stufe a) die Aminoschutzgruppe Boc ist.

**3.** Verfahren zur Gewinnung einer Peptidzusammensetzung nach Anspruch 1, worin in Stufe b) die halogenierten Phenolderivate unter den fluorierten und chlorierten Derviaten ausgewählt werden.

**4.** Verfahren nach Anspruch 3, worin die halogenierten Phenolderivaten Pentachlorphenol, Trichlorphenol und Pentafluorphenol sind.

**5.** Verfahren zur Gewinnung einer Peptidzusammensetzung nach Anspruch 1, worin in Stufe b) das Molverhältnis zwischen dem Tetrapeptid (II) und dem halogenierten Phenolderivat 1:1 beträgt oder nahe diesem Wert liegt und die Umsetzung in flüssiger Phase in einem inerten organischen Lösungsmittel bei einer Temperatur von -10° bis +40°C ausgeführt wird.

**6.** Verfahren nach Anspruch 5, worin das organische Lösungsmittel Ethylacetat ist.

**7.** Verfahren nach Anspruch 5, worin die Temperatur zwischen 0° und 25°C liegt.

**8.** Verfahren zur Gewinnung einer Peptidzusammensetzung nach Anspruch 1, worin in Stufe c) die Aminoschutzgruppe durch Acidolyse mit Trifluoressigssäure oder Chlorwasserstoffsäure bei Raumtemperatur (20 bis 25°C) während einer Zeitspanne von einer Stunde oder nahezu einer Stunde abgetrennt wird.

**9.** Verfahren zur Gewinnung einer Peptidzusammensetzung nach Anspruch 1, worin in Stufe d) das tertiäre Alkylamin Triethylamin ist.

**10.** Verfahren nach Anspruch 9, worin die Polykondensation bei Raumtemperatur (20 bis 25°C) oder nahe Raumtemperatur ausgeführt wird.

**11.** Verfahren zur Gewinnung einer Peptidzusammensetzung nach Anspruch 1, worin in Stufe e) die Auftrennung durch Gelchromatographie ausgeführt wird.

**12.** Verwendung der Zusammensetzung nach Anspruch 1 zur Herstellung von Malaria-Impfstoffen.

**13.** Verwendung der Zusammensetzung nach Anspruch 1 zur Herstellung von Diagnosekits zur Feststellung von Malaria-Erkrankungen.

**14.** Verfahren zur Feststellung von für Plasmodium falciparum-Sporozoiten spezifischen Antikörpern in Humanblut nach einem ELISA-Test unter Verwendung einer synthetischen Peptidzusammensetzung nach Anspruch 1 als Substrat.

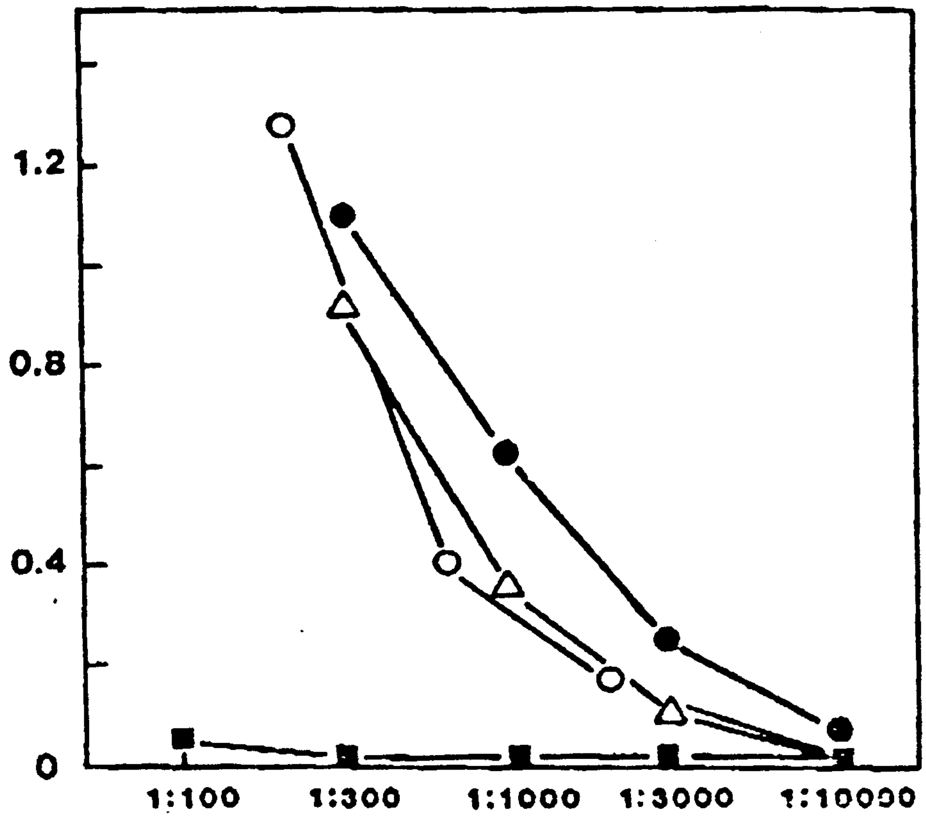


FIGURA 1